# EUROPEAN PATENT SPECIFICATION

(11) **EP 0 588 124 B1**
(45) Date of publication and mention of the grant of the patent: **20.01.1999**
(21) Application number: 93113739.2
(22) Date of filing: 27.08.1993
(51) Int. Cl.: A61N 1/39, A61N 1/365

(54) **Heart defibrillator**
Vorrichtung zur Defibrillation des Herzens
Appareil defibrillation cardiaque

(30) Priority: 16.09.1992 SE 9202665
(43) Date of publication of application: 23.03.1994
(73) Proprietor: Pacesetter AB, 175 84 Järfälla (SE)
(72) Inventor: Hedberg, Sven-Erik, S-19632 Kungsängen (SE)
(74) Representative: Harrison, Michael Charles

(56) References cited:
- EP-A- 0 540 266
- US-A- 4 559 946
- US-A- 4 800 883
- US-A- 5 111 813

## Description

The present invention relates to a heart defibrillator comprising a defibrillation circuit for delivering defibrillation pulses

Complete therapeutic devices for stimulating the heart have previously been disclosed in e.g. US-A 4,407,288 and EP-A-0 253 505. These devices are devised to emit electrical impulses of varying strength and frequency for various pacemaker functions, cardioversion or heart defibrillation.

US-A-4 800 883 discloses a heart defibrillor which is capable of generating single pulses having a multiphasic waveform. US-A-5 111 813 discloses a heart defibrillator in which the energy delivered in the defibrillation pulse is adjusted on the basis of a prior measurement of impedance. EP-A-0 540 266 belongs to the state of the art only by virtue of Article 54(3) EPC. This document discloses a heart defibrillator which also generates pre-pulses. It does not disclose an impedance measurement circuit which measures the impedance situation between the defibrillation electrodes after emission of the pre-pulse in order to control the defibrillation circuit so a defibrillation pulse is emitted for a specific, predesignated, measured impedance situation.

The object of the present invention is to produce a heart defibrillator making possible effective heart defibrillation with the expenditure of less energy than has hitherto been possible.

This object is achieved with a heart defibrillator of the above-described kind with the features specified in claim 1.

When a pre-pulse, not as intense as the latter but still intense enough to induce mechanical contraction of muscles in parts of the thorax and possibly the heart, is emitted before the actual defibrillation pulse, the heart and the thorax are geometrically affected, and the heart's electrical conductivity should change, thereby enhancing the effect of the subsequent defibrillation pulse. The pre-pulse achieves mechanical preparation of the heart before the defibrillation pulse is emitted.

According to advantageous embodiments of the defibrillator according to the invention, the pre-pulse and the defibrillation pulse can be emitted between the same or different electrodes, the electrodes being selected for the pulses such that the current distribution and the current density in myocardium is optimal for the defibrillation pulse. Even the time at which the defibrillation pulse is emitted after the pre-pulse is selected so an optimal effect is achieved by determining the time automatically with the aid of an impedance measurement circuit which measures the impedance between the defibrillation electrodes after the pre-pulse and which controls the defibrillation circuitry so the defibrillation pulse is emitted when the impedance reaches a specific, optimal value. This optimal impedance situation is an impedance situation at which successful defibrillation from experience is known to be most likely.

As an example, one embodiment of the heart defibrillator according to the invention will now be more fully described with reference to the appended drawings, in which
FIG. 1 shows an example of a pre-pulse and a defibrillation pulse emitted by the heart defibrillator according to the invention;
FIG. 2 shows the general structure, in block diagram form, of the heart defibrillator according to the invention;
FIG. 3 shows a block diagram of the impedance measurement circuit in the heart defibrillator according to the invention, and
FIG. 4 shows a therapeutic device, implanted with a number of electrodes in and around the heart of a patient, for heart stimulation.

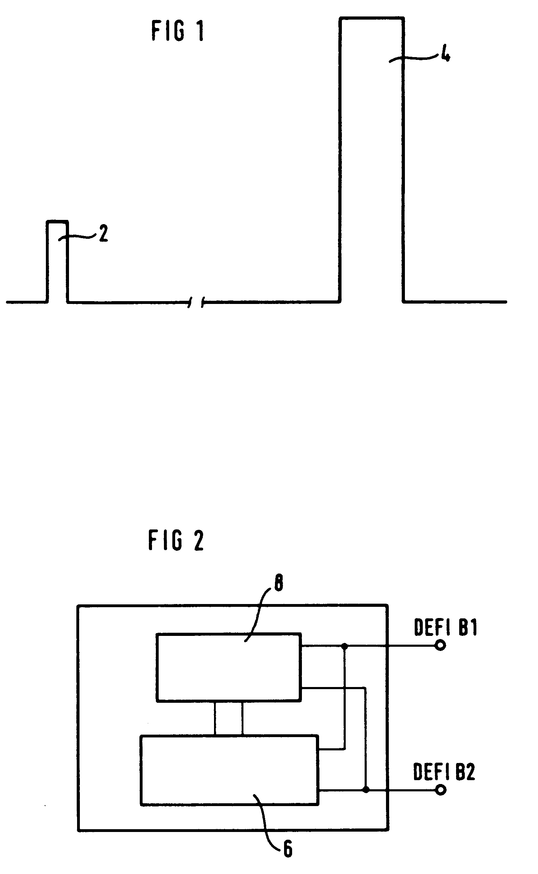

FIG. 1 shows a pre-pulse 2 which is emitted by the heart defibrillator according to the invention before the actual defibrillation pulse 4. The pre-pulse is strong enough to induce mechanical contraction of the muscles in parts of the thorax and possibly the heart. Thus, tissue in the heart contracts, achieving a kind of mechanical preparation of the heart, thereby improving the subsequent defibrillation pulse's 4 likelihood of attaining the desired defibrillation of the heart.

The energy of the pre-pulse is accordingly much less than the energy in the defibrillation pulse. Since the heart is prepared by the pre-pulse, the energy of the subsequent defibrillation pulse can be reduced, compared to the situation with prior art defibrillators, while still reliably producing effective defibrillation of the heart. Thus, effective heart defibrillation with the expenditure of less total energy is possible with a defibrillator according to the invention.

The defibrillator according to the invention comprises a defibrillation circuit 6 which emits pre-pulses 2 and defibrillation pulses 4 across electrode contacts DEFIB 1 and DEFIB 2. The defibrillation circuit 6 contains a control unit in the form of a microprocessor (not shown) for control purposes.

The defibrillator according to the invention further comprises an impedance measurement circuit 8 which, after the pre-pulse 2, continuously measures the impedance situation between the defibrillation electrodes. The impedance measurement circuit 8 is also controlled by the microprocessor in the defibrillation circuit 6. Thus, the impedance measurement is started and stopped over a first port on the microprocessor, whereas a second port on the microprocessor reads the obtained and A/D-converted impedance signal. See below!

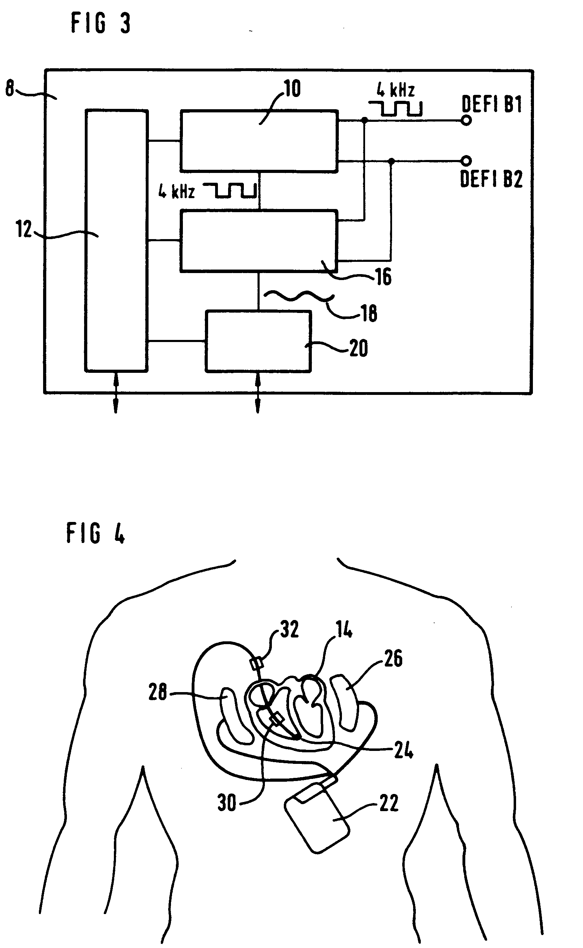

The impedance measurement circuit 8 comprises a current generator 10, which is induced, over a logic control unit 12, by the microprocessor in the defibrillation circuit to emit a measurement current with a frequency of 4 kHz (cf. FIG. 3). This measurement current is emitted across contacts DEFIB 1 and DEFIB 2 of the defibrillation electrodes.

A measurement signal, also with a measurement frequency of 4 KHz and with varying, impedance dependent amplitude, is received, and a signal synchronous with the measurement current and the received measurement signal are sent to a synchronous demodulator 16. The output signal from this demodulator 16 consists of a low-frequency signal representing the impedance variation measured across contacts DEFIB 1 and DEFIB 2. This impedance signal is illustrated at 18 in FIG. 3.

The impedance signal 18 is fed to the A/D converter 20 and then sent to the microprocessor in the defibrillation circuit 6.

The A/D converted signal is stored in the defibrillation circuit 6 and analyzed by the microprocessor according to a special program for establishing the optimal time for emission of the defibrillation pulse 4. Thus, the defibrillation pulse is emitted when a predesignated optimal impedance situation is detected.

FIG. 4 schematically shows a plurality of different heart electrodes 22, 24, 26, 28, 30, 32 arranged in and around the heart 14. Here, the device case 22 can also serve as an electrode, and so-called patches are shown arranged on either side of the heart 14 at 26 and 28. The pre-pulse 2 and the defibrillation pulse 4 can be optionally emitted either by the same or different electrodes. Both the defibrillation electrodes and the time for the defibrillation pulse are selected so current distribution and current density become optimal in myocardium and so the largest possible part of the heart is subjected to the defibrillation shock.

One embodiment has been described above in which the time for emission of the defibrillation pulse is established by impedance monitoring, whereby the defibrillation pulse is emitted when a predesignated, optimal impedance situation is detected between the defibrillation electrodes. The impedance normally drops after the pre-pulse, and the predesignated impedance situation for emission of the defibrillation pulse can then be based on the drop in the impedance below a predesignated level. The time elapsing between the pre-pulse and the defibrillation pulse may be controlled to be within a maximum of 0.5 s.

The heart defibrillator according to the invention can further be devised as an implantable or external defibrillator.

## Claims

1. A heart defibrillator comprising a defibrillation circuit (6) for delivering defibrillation pulses, and a plurality of electrodes (22, 24, 26, 28, 30, 32) connected to the defibrillation circuit (6), the defibrillation circuit (6) being devised to emit a pre-pulse (2) before a defibrillation Pulse (4) of lower energy than the latter, said pre-pulse (2) having enough energy to induce mechanical contraction of muscles in parts of the thorax and possibly in the heart and to induce a drop in the impedance between the defibrillation electrodes, the heart defibrillator further comprising an impedance measurement circuit (8) which measures the impedance situation between the defibrillation electrodes (22, 26, 28, 30, 32) after emission of the pre-pulse (2) in order to control the defibrillation circuit (6) so a defibrillation pulse (4) is emitted for a specific, predesignated, measured impedance situation.

2. The defibrillator of claim 1, characterized in that the defibrillation circuit (6) is devised to emit pre-pulse (2) and defibrillation pulse (2) between the same electrodes (22, 26, 28, 30, 32).

3. The defibrillator of claim 1, characterized in that the defibrillation circuit (6) is devised to emit pre-pulse (2) and defibrillation pulse (4) between different electrodes (22, 26, 28, 30, 32).

4. The defibrillator of any of claims 1 - 3, characterized in that the defibrillation circuit (6) is controlled so it emits the defibrillation pulse (4) within a maximum of 0.5 s after the pre-pulse (2).

5. The defibrillator of claim 1, characterized in that the impedance measurement circuit (8) comprises a current generator (10) which sends a measurement current to the defibrillation electrodes (22, 26, 28, 30, 32) and a synchronous demodulator (16) which is devised to receive a signal with the same phase as the measurement current and a signal with an amplitude dependent on the impedance between the defibrillation electrodes (22, 26, 28, 30, 32).

6. The defibrillator of claim 5, characterized in that the demodulator (16) is devised to send a low-frequency output signal, representing the variation in the impedance between the defibrillation electrodes (22, 26, 28, 30, 32), to an A/D converter (20) which sends its output signal to the defibrillation circuit (6) in order to control the same.

7. The defibrillator of claim 6, characterized in that the defibrillation circuit (6) comprises a control unit, preferably a microprocessor, which also controls the impedance measurement circuit (8), and in that the signal from the A/D converter (20) is analyzed by the control unit of the defibrillation circuit (6), and the defibrillation pulses (4) are emitted after measurement of the predesignated impedance situation.

8. The defibrillator of any of claims 1 or 5-7, characterized in that the impedance measurement circuit (8) is arranged to control the defibrillation circuit (6) so a defibrillation pulse (4) is emitted when the impedance between the defibrillation electrodes (22, 26, 28, 30, 32) has dropped to a predesignated value.

## Patentansprüche

1. Ein Herzdefibrillator mit einer Defibrillationsschaltung (6) zur Abgabe von Defibrillationsimpulsen und einer Vielzahl von mit der Defibrillationsschaltung (6) verbundener Elektroden (22, 24, 26, 28, 30, 32), wobei die Defibrillationsschaltung (6) dazu ausgebildet ist, vor einem Defibrillationsimpuls (4) einen Vorimpuls (2) niedrigerer Energie als die des nachfolgenden Impulses abzugeben, wobei der Vorimpuls (2) genügend Energie hat, um eine mechanische Kontraktion der Muskeln in Teilen des Thorax und möglicherweise des Herzens auszulösen und ein Absinken der Impedanz zwischen den Defibrillationselektroden auszulösen, und wobei der Herzdefibrillator weiterhin eine Impedanzmeßschaltung (8) aufweist, die die Impedanzsituation zwischen den Defibrillationselektroden (22, 26, 28, 30, 32) nach der Abgabe des Vorimpulses (2) mißt, um die Defibrillationsschaltung (6) so zu steuern, daß ein Defibrillationsimpuls (4) bei einer spezifischen, vorbestimmten, gemessenen Impedanzsituation abgegeben wird.

2. Der Defibrillator nach Anspruch 1, **dadurch gekennzeichnet,** daß die Defibrillationsschaltung (6) dazu ausgebildet ist, Vorimpulse (2) und Defibrillationsimpulse (2) zwischen den selben Elektroden (22, 26, 28, 30, 32) abzugeben.

3. Der Defibrillator nach Anspruch 1, **dadurch gekennzeichnet,** daß die Defibrillationsschaltung (6) dazu ausgebildet ist, Vorimpulse (2) und Defibrillationsimpulse (22, 26, 28, 30, 32) zwischen verschiedenen Elektroden abzugeben.

4. Der Defibrillator nach einem der Ansprüche 1 - 3, **dadurch gekennzeichnet,** daß die Defibrillationsschaltung (6) so gesteuert wird, daß sie den Defibrillationsimpuls (4) innerhalb eines Maximums von 0.5 s nach dem Vorimpuls (2) abgibt.

5. Der Defibrillator nach Anspruch 1, **dadurch gekennzeichnet,** daß die Impedanzmeßschaltung (8) einen Stromgenerator (10), der einen Meßstrom an die Defibrillationselektroden (22, 26, 28, 30, 32) liefert, und einen synchronen Demodulator (16), der dazu ausgebildet ist, ein Signal mit der selben Phase wie der Meßstrom und ein Signal mit einer von der Impedanz zwischen den Defibrillationselektroden (22, 26, 28, 30, 32) abhängenden Amplitude zu empfangen, aufweist.

6. Der Defibrillator nach Anspruch 5, **dadurch gekennzeichnet,** daß der Demodulator (16) dazu ausgebildet ist, ein niederfrequentes Ausgangssignal, das die Variation in der Impedanz zwischen den Defibrillationselektroden (22, 26, 28, 30, 32) repräsentiert, an einen A/D-Wandler (20) zu senden, der sein Ausgangssignal an die Defibrillationsschaltung (6) sendet, um dieselbe zu steuern.

7. Der Defibrillator nach Anspruch 6, **dadurch gekennzeichnet,** daß die Defibrillationsschaltung (6) eine Steuereinheit aufweist, vorzugsweise einen Mikroprozessor, der auch die Impedanzmeßschaltung (8) steuert, und daß das Signal von dem A/D-Wandler (20) durch die Steuereinheit der Defibrillationschaltung (6) analysiert wird und die Defibrillationsimpulse (4) nach der Messung der vorbestimmten Impedanzsituation abgegeben werden.

8. Der Defibrillator nach einem der Ansprüche 1 oder 5 - 7, **dadurch gekennzeichnet,** daß die Impedanzmeßschaltung (8) dazu eingerichtet ist, die Defibrillations-Schaltung (6) so zu steuern, daß ein Defibrillationsimpuls (4) abgegeben wird, wenn die Impedanz zwischen den Defibrillationselektroden (22, 26, 28, 30, 32) um einen vorbestimmten Wert abgesunken ist.

## Revendications

1. Défibrillateur cardiaque comportant un circuit (6) de défibrillation destiné à envoyer des impulsions de défibrillation, et une pluralité d'électrodes (22, 24, 26, 28, 30, 32) reliées au circuit (6) de défibrillation, le circuit (6) de défibrillation étant conçu pour émettre une pré-impulsion (2) avant une impulsion (4) de défibrillation d'énergie plus faible que l'énergie de cette dernière, la pré-impulsion (2) ayant suffisamment d'énergie pour induire une contraction mécanique des muscles dans des parties du thorax et éventuellement dans le coeur et pour induire une chute de l'impédance entre les électrodes de défibrillation, le défibrillateur cardiaque comportant en outre un circuit (8) de mesure d'impédance qui mesure la situation d'impédance entre les électrodes (22, 26, 28, 30, 32) après l'émission de la pré-impulsion (2) afin de commander le circuit (6) de défibrillation de sorte qu'une impulsion (4) de défibrillation soit émise pour une situation d'impèdance mesurée préconçue précise.

2. Défibrillateur suivant la revendication 1, caractérisé en ce que le circuit (6) de défibrillation est conçu pour émettre une pré-impulsion (2) et une impulsion (2) de défibrillation entre les mêmes électrodes (22, 26, 28, 30, 32) .

3. Défibrillateur suivant la revendication 1, caractérisé en ce que le circuit (6) de défibrillation est conçu pour émettre une pré-impulsion (2) et une impulsion (4) de défibrillation entre des électrodes (22, 26, 28, 30, 32) différentes.

4. Défibrillateur suivant l'une quelconque des revendications 1 à 3, caractérisé en ce que le circuit (6) de défibrillation est commandé de sorte qu'il émet l'impulsion (4) de défibrillation à l'intérieur d'un maximum de 0,5 seconde après la pré-impulsion (2).

5. Défibrillateur suivant la revendication 1, caractérisé en ce que le circuit (8) de mesure d'impédance comprend un générateur (10) de courant qui envoie un courant de mesure aux électrodes (22, 26, 28, 30, 32) de défibrillation et un démodulateur (16) synchrone qui est conçu pour recevoir un signal ayant la même phase que le courant de mesure et un signal ayant une amplitude fonction de l'impédance entre les électrodes (22, 26, 28, 30, 32) de défibrillation.

6. Défibrillateur suivant la revendication 5, caractérisé en ce que le démodulateur (16) est conçu pour envoyer un signal de sortie basse fréquence, représentant la variation de l'impédance entre les électrodes (22, 26, 28, 30, 32) de défibrillation à un convertisseur (20) analogique/numérique (A/N) qui envoie son signal de sortie au circuit (6) de défibrillation afin de commander celui-ci.

7. Défibrillateur suivant la revendication 6, caractérisé en ce que le circuit (6) de défibrillation comprend une unité de commande, de préférence un microprocesseur, qui commande également le circuit (8) de mesure d'impédance et en ce que le signal provenant du convertisseur (20) analogique/numérique (A/N) est analysé par l'unité de commande du circuit (6) de défibrillation, et les impulsions (4) de défibrillation sont émises après mesure de la situation d'impédance préconçue.

8. Défibrillateur suivant l'une quelconque des revendications 1 ou 5 à 7, caractérisé en ce que le circuit (8) de mesure d'impédance est agencé pour commander le circuit (6) de défibrillation de sorte qu'une impulsion (4) de défibrillation est émise lorsque l'impédance entre les électrodes (22, 26, 28, 30, 32) de defibrillation est tombée à une valeur préconçue.
